# EUROPÄISCHE PATENTANMELDUNG

(11) **EP 1 146 129 A2**
(43) Veröffentlichungstag der Anmeldung: **17.10.2001**
(21) Anmeldenummer: 01107541.3
(22) Anmeldetag: 27.03.2001
(51) Int. Cl.: C12Q 1/68

(54) **Verfahren zum Auffinden von Oligonukleotid-Sequenzen für Nukleinsäure-Amplifikationsverfahren**

(30) Priorität: 14.04.2000 DE 10018901; 19.05.2000 DE 10024830
(71) Anmelder: Aventis Behring GmbH, 35002 Marburg (DE)
(72) Erfinder: Weimer, Thomas, Dr., 35075 Gladenbach (DE)

(57) **Zusammenfassung**

Es wird ein Verfahren zum Auffinden von heterologen Oligonukleotid-Sequenzen für ein Nukleinsäure-Amplifikationsverfahren beschrieben, bei dem
a) durch Fragmentierung konservierter Bereiche der zu amplifizierenden Zielnukleinsäure sich gegenseitig überlappende Oligonukleotid-Sequenzen erzeugt,
b) diese Sequenzfragmente zum Auffinden ähnlicher DNA-Abschnitte in der Genbank oder anderen DNA-Datenbanken benutzt und damit geeignete heterologe Oligonukleotid-Sequenzen artfremder Organismen ermittelt, und
c) die aufgefundenen heterologen Oligonukleotid-Sequenzen als Primer und/oder Sonden zur Gewinnung der Zielnukleinsäure nach einem Nukleinsäure-Amplifikationsverfahren eingesetzt werden.

## Beschreibung

Gegenstand der Erfindung ist ein Verfahren zum Auffinden von heterologen Oligonukleotid-Sequenzen, die zum Nachweis einer speziellen, vorbestimmten und genau bekannten Zielnukleinsäure einschließlich unbekannter Varianten und Mutanten dieser Zielnukleinsäure geeignet sind.

Nukleinsäure-Amplifikationsverfahren (NAT) wie z.B. PCR (Polymerasekettenreaktion), NASBA (=Nucleic Acid Sequence-Based Amplification), TMA (Transcription Mediated Amplification), LCR (Ligase Chain Reaction) und andere sind effektive Methoden, um in vitro große Mengen einer spezifischen DNA-Sequenz anzureichern und damit der Analyse zugänglich zu machen. Da jeder beliebige DNA-Abschnitt vervielfältigt werden kann, haben diese Methoden, allen voran die PCR, vielfältige Anwendungen gefunden und dienen u.a. auch zum Nachweis viraler Verunreinigungen, die in Blut oder Blutplasma enthalten sein können. Um die Virussicherheit von Plasmaprodukten zu erhöhen, hat sich deshalb ihre Untersuchung auf Virusnukleinsäuren mittels NAT eingebürgert. In der Virus-Diagnostik können damit Virus-Genome direkt im Patientenblut aufgefunden werden, noch bevor virale Proteine oder Antikörper dagegen im Blut nachweisbar sind.

Die PCR basiert darauf, dass drei Reaktionsschritte vielfach wiederholt ablaufen: Der Reaktionsansatz mit Doppelstrang-DNA mit der gesuchten Sequenz als Matritze wird durch Hitze denaturiert und in die beiden Einzelstränge aufgespalten. Beim Abkühlen hybridisieren die im Überschuss zugesetzten Primer mit den komplementären Basen-Sequenzen auf der Matritzen-DNA. Als Primer werden dabei synthetische Oligonukleotide mit 15 bis 30 Basen eingesetzt, die zum Anfang und zum Ende des gesuchten DNA-Abschnitts komplementäre Sequenzen aufweisen. Der gesuchte DNA-Abschnitt ist damit durch die beiden Primer flankiert. Beim dritten Reaktionsschritt wird die Temperatur auf das Optimum der hitzestabilen DNA-Polymerase gebracht. Ausgehend von den Primern baut die Polymerase eine Kopie pro Ausgangs-DNA auf, wobei die Länge der zu duplizierenden DNA durch den Abstand zwischen den Primern bestimmt ist. Durch mehrfache Wiederholung dieser Prozessschritte wird die Ziel-DNA amplifiziert und der Analyse zugänglich gemacht.

Voraussetzung für eine zielgerichtete Durchführung einer PCR sind Primer-Sequenzen, die mit jeweils einem der DNA-Stränge auf beiden Seiten des zu amplifizierenden DNA-Abschnitts hybridisieren. Deshalb ist eine genaue Kenntnis der Nukleotid-Sequenzen am Beginn und am Ende des zu duplizierenden DNA-Abschnitts erforderlich. Zur Herstellung geeigneter Primer wird es deshalb bisher als erforderlich angesehen, einen zur selektiven Hybridisierung geeigneten Primer herzustellen, dessen ausreichende Hybridisierung mit dem Untersuchungsmaterial dadurch sichergestellt wird, dass er eine artspezifische, d.h. autologe Nukleotid-Sequenz aufweist.

Selektive Hybridisierung in diesem Zusammenhang heißt, dass ein solcher, selektiv hybridisierender Primer nur und ausschließlich mit dem nachzuweisenden DNA-Abschnitt, d.h. der Zielnukleinsäure hybridisiert.

Es hat sich nun allerdings gezeigt, dass das herkömmliche PCR-Verfahren durch die Festlegung auf artspezifische, autologe Primer und auf die bei diesem Verfahren ebenfalls eingesetzten autologen Oligonukleotid-Sonden in seiner Anwendungsbreite limitiert und gelegentlich nicht geeignet ist, unbekannte Varianten der nachzuweisenden DNA-Sequenz zu erfassen.

Es stellte sich deshalb die Aufgabe, die Anwendungsbreite von Nukleinsäure-Amplifikationsverfahren, besonders des PCR-Verfahrens zu verbessern, indem eine Auswahl zur Zielnukleinsäure nicht-selektiv hybridisierender Primer zum Nachweis dieser Zielnukleinsäure zur Verfügung gestellt wird. Gelöst wird diese Aufgabe durch ein Verfahren zur Gewinnung heterologer, zur Zielnukleinsäure nicht-selektiv hybridisierender Primer aus bezüglich der Zielnukleinsäure fremden Organismen, wobei diese Primer dennoch zur Amplifikation einer Zielnukleinsäure geeignet sind.

Gegenstand der Erfindung ist deshalb ein Verfahren zum Auffinden von heterologen Oligonukleotid-Sequenzen für ein Nukleinsäure-Amplifikationsverfahren, bei dem
a) durch Fragmentierung konservierter Bereiche der zu amplifizierenden Nukleinsäure sich gegenseitig überlappende, vorzugsweise 30 bis 50 Basen umfassende Sequenzfragmente (z.B. 1 bis 50, 25 bis 75, 50 bis 100, usw.) erzeugt,
b) diese Sequenzfragmente zum Auffinden ähnlicher DNA-Abschnitte in der Genbank oder anderen DNA-Datenbanken, z.B. EMBL, benutzt und damit heterologe, d.h. hybridisierende Oligonukleotid-Sequenzen artfremder Organismen ermittelt, und
c) die aufgefundenen heterologen, hybridisierenden Oligonukleotid-Sequenzen als Primer und/oder Sonden zur Gewinnung der Zielnukleinsäure nach einem Nukleinsäure-Amplifikationsverfahren eingesetzt werden.

Dieses Verfahren kann mit besonderem Vorteil zum Nachweis von viralen Sequenzabschnitten verwendet werden, indem durch Fragmentierung vorzugsweise konservierter Bereiche des Genoms eines Virus sich gegenseitig überlappende Sequenzfragmente erzeugt und in einer Genbank damit hybridisierende Oligonukleotid-Sequenzen artfremder Organismen ermittelt werden. Die Sequenzfragmente sollten vorzugsweise 30 bis 50 Basen umfassen.

Die Aufspaltung erfolgt aufgrund der Beobachtung, dass viele Homologiesuchprogramme (wie z.B. FastN, Blast oder Wordsearch des Wisconsin Packages der Genetics Computer Group Inc.) darauf ausgelegt sind, Sequenzähnlichkeiten oder - homologien zu kompletten Genen oder größeren DNA-Sequenzen zu finden, die Aufgabe bei der Suche nach für Nukleinsäuretechnologien geeigneten Primern und Probes aber gerade darin besteht, kurze Sequenzen mit sehr hoher Ähnlichkeit aufzufinden. Auch empfiehlt es sich, die Zielvirus-Sequenzen von der Homologiesuche von vornherein auszuschließen, um die Erfolgsaussicht auf möglichst viele heterologe Sequenzen zu erhöhen.

Die bei den beschriebenen Homologieuntersuchungen in einer Genbank aufgefundenen Sequenzen zeigen unterschiedliche Homologiegrade und Sequenzlängen, die von den eingesetzten, fragmentierten Oligonukleotid-Sequenzen abweichen können. Diese heterologen Oligonukleotid-Sequenzen müssen deshalb anschließend noch einmal sorgfältig auf ihre Eignung als Primer und Sonden überprüft werden. Wichtige Kriterien für die Eignung der heterologen als Ersatz für die autologen Primer und Sonden sind die Gesamtlänge der homologen Sequenz, die Anzahl der aufeinanderfolgenden Nukleotide, die vorhandene Anzahl der Fehlstellen (mismatches), ihr G/C-Gehalt und die berechnete Denaturierungstemperatur als Maß für die Bindungsfestigkeit des Primers oder der Sonde an der DNA.

Enthalten die so gewonnenen heterologen Primer noch Fehlstellen (mismatches), dann können die dort stehenden Basen durch eine "Universalbase" wie z.B. Inosin ersetzt werden, wodurch eine vollständige Hybridisierung der Nukleotid-Sequenz des heterologen Primers mit der Zielnukleinsäure erreicht wird.

Mit diesem Verfahren lassen sich somit aus artfremden Organismen heterologe Primer gewinnen, die in gleicher Weise zur Hybridisierung mit der Zielnukleinsäure geeignet sind wie autologe Primer, d.h. Primer, die aus den DNA-Sequenzen des Organismus abgeleitet sind, der die Zielnukleinsäure enthält. Die so gewonnenen heterologen Oligonukleotide können auch zur Herstellung einer für eine PCR einsetzbaren Sonde benutzt werden. Derartige Sonden sind häufig fluoreszenzmarkiert und basieren z.B. auf einem 5'-Nuklease-Assay (Livak et al. 1995. Oligonucleotides with fluorescent dyes at opposite ends provide a quenched probe system useful for detecting a PCR product and nucleic acid hybridization. PCR Meth. Applic. 4:357-362) oder auf einer besonderen Sekundärstruktur (Tyagi et al. Molecular Beacons: Probes that Fluoresce upon Hybridization. Nature Biotechnology. March 1996, Vol. 14, pages 303-308).

Es ist aber auch möglich, für das erfindungsgemäße Verfahren einen Primer einzusetzen, der mit zwei Fluoreszenzfarbstoffen (Reporter und Quencher) markiert ist, wobei dieser Primer mit der zu amplifizierenden DNA-Sequenz am 3'-Ende nicht vollständig hybridisiert. Dieses Verfahren ist in der deutschen Patentanmeldung 197 55 642.6 beschrieben. Bei der Amplifikation, für die eine oder mehrere thermostabile DNA-Polymerasen eingesetzt werden können, von denen wenigstens eine auch Korrektureigenschaften (proof-reading) haben muss, werden dann die nicht gepaarten Basen des markierten Primers zusammen mit dem daran befestigten Quencherfarbstoff freigesetzt, wodurch die Fluoreszenz auf der Wellenlänge des Reporterfarbstoffes ansteigt.

Die nach dem erfindungsgemäßen Verfahren gewonnenen und für eine PCR einsetzbaren heterologen Oligonukleotide zeichnen sich also dadurch aus, dass sie als Primer oder Sonden nicht nur mit der DNA-Sequenz der Zielnukleinsäure, sondern auch mit Nukleinsäuren artfremder Organismen hybridisieren. Trotzdem eignen sie sich zum Nachweis oder zur Gewinnung der Zielnukleinsäure genauso wie autologe, d.h. aus dem gleichen Organismus abgeleitete Oligonukleotide.

Nach oben beschriebener Vorgehensweise wurde die konservierte 5'-nicht translatierte Region des Hepatitis C-Virus (HCV) zum Auffinden heterologer Sequenzen benutzt. Die folgenden Sequenzen sind ein Ausschnitt der Suchergebnisse und wurden zum Nachweis von HCV mittels PCR benutzt.

Die SEQ IDs zeigen zunächst die abgeleitete Primer-(SEQ IDs 1, 2, 3, 4, 6, 7, 8) oder Sondensequenzen (5, 9), darunter die Homologie des jeweiligen HCV-Bereichs zur heterologen Sequenz. In den verwendeten Sequenzen wurden die nicht homologen Basen durch Inosin (I) ersetzt. 
SEQ ID No. 1:

5' GGT ICA IGG TCT AIG AGA CII CCC GGG3'
AB007366 Red Sea Bream Iridovirus gene for DNA polymerase

345 ..TCATGGTGCACGGTCTACGAGACCTCCCGGG... 315
||| || |||||| |||||  ||||||
1184 AGCATGGGTTCAGGGTCTATGAGACGCCCCGGGCGT 1219
 wobei "^{5'} GGT ICA IGG TCT AIG AGA CII CCC GGG ^{3'}" die Sequenz eines Primers darstellt. AB007366 ist die Accession Number in der GenBank, unter der die Sequenz des DNA Polymerase Gens des Red Sea Bream Iridovirus abgelegt ist. Der Sequenzvergleich zeigt die Homologie zwischen HCV (oben) und des DNA Polymerase Gens (unten), wobei ein | für eine identische Base steht.

SEQ ID No. 2:

5' ACT CCA CCA TAG ATC ACT 3'

AB020864 Homo sapiens genomic DNA of 8p21.3-p22

31 GGAGTGATCTATGGTGGAGT 12
| ||||||||||||||||||
95982 GCAGTGATCTATGGTGGAGT 96001

SEQ ID No. 3:

5' CTA ICC ATG GCI TTA GTA TGA G 3'

AC004616 Homo sapiens Xp22

88 CTCATACTAACGCCATGGCTAG 67
|||||||||| ||||||| |||
80839 CTCATACTAAAGCCATGGATAG 80860

SEQ ID No. 4:

5' AGC ACC CTI TCA GGC AGT ACC 3'

Z97055 Human DNA sequence from PAC 388M5 on chromosome 22

285 .GGTACTGCCTGATAGGGTGCTTGCGAGTGCC ... 315
     |||||||||||| ||||||||    |   |
50941 TGGTACTGCCTGAGAGGGTGCTGCTGCCTTTGGGA 50975

SEQ ID No. 5:

5' FAM-TGG GTC  ICG  AAA  GIC  CTT  GT-TAMRA 3'

AJ009757 Helianthus tuberosus sst-1 gene

274  CCACAAGGCCTTTCGCGACCCAAC 251
     | |||||| |||||| |||||| |
711  CTACAAGGACTTTCGGGACCCATC 734

SEQ ID No. 6:

5' GCT CAT GIT GCA CGI ICT ICG AGA C 3'

AJ010298 Drosophila melanogaster retrotransposon-like element

335 GCTCATGATGCACGGTCTACGAGAC 311
    ||||||| ||||||  || ||||||
4557 GCTCATGGTGCACGAGCTCCGAGAC 4581

SEQ ID No. 7:
5' CAT AGI TCA CTC CCC TGT GA 3'

AF111207 Cyprinella galactura NADH dehydrogenase subunit 2 (ND2)
gene

60 .CAGTAGTTCCTCACAGGGGAGTGATCTATGG... 30
     |   ||   |||||||||||||| |||||
228 CAATGCGTGGATCACAGGGGAGTGAACTATGACTA 262

60 ...AAAGCGTCTAGCCATGGCGTTAGTATGATG 89
       |||| | ||||||||| | |||||| ||
92 CGAAAAGAGGCTAGCCATGCCCTTAGTAGGACT 124

SEQ ID No. 9:

5' FAM-GTA CCT GGG TCI CGA AAG ICC TTG TGG TAC T-TAMRA 3'

AJ009757 Helianthus tuberosus sst-1 gene

274 CCACAAGGCCTTTCGCGACCCAAC 251
| |||||| |||||| |||||| |
711 CTACAAGGACTTTCGGGACCCATC 734

Setzt man die vorstehend genannten Primer und Sonden für eine PCR ein, dann kann eine Nukleinsäure-Amplifikation nur bei Anwesenheit der Hepatitis C Virusnukleinsäure stattfinden, weil Voraussetzung für die PCR ist, dass zumindest ein Primerpaar mit der zu amplifizierenden Nukleinsäure hybridisiert. Das aber ist im vorliegenden Fall nur bei Anwesenheit von Hepatitis C Virus möglich, denn für keine andere Nukleinsäure steht das erforderliche Primerpaar zur Verfügung. Eine nested PCR erhöht hier weiterhin die Spezifität.

Die nachfolgenden Reaktionsansätze wurden dazu benutzt, die Spezifität und Sensitivität der beschriebenen Primer und Sonden bezüglich eines HCV-Nachweises zu demonstrieren.

### Beispiel 1: HCV nested PCR, TaqMan Detektion

### RNA Extraktion

Zum Nachweis von Hepatitis C Virus RNA wird diese zunächst mit Hilfe von Standardverfahren extrahiert (z.B. Ishizawa M., Kobayashi Y., Miyamura T., Matsuma, S: Simple procedure of DNA isolation from human serum. Nucl. Acids Res. 1991; 19:5792). Die Amplifikation wird wie folgt angesetzt:

### cDNA Synthese

Zehn µl der extrahierten RNA werden mit 4 µl 5x First Strand Buffer (Life Technologies), 2 µl des Primers 1 (50 pMol/µl; siehe SEQ ID No. 1 des Sequenzprotokolls), 1 µl dNTPs (10 mM), 2 µl Dithiothreitol (100 mM), 0.75 µl Wasser und 0,25 µl Superscript (50 Units, Life Technologies) gemischt und eine Stunde bei 42° C inkubiert. Anschließend erfolgt die Enzyminaktivierung für 5 Minuten bei 95° C.

### 1. PCR

Zum cDNA Ansatz werden 80µl eines 1.PCR-Reaktionsgemisches [61.7µl Wasser, 8µl 10x PCR-Puffer (Perkin Elmer), 2µl des Primers 2 (50pmol/µl; siehe SEQ ID No. 2 des Sequenzprotokolls), 4.8µl Magnesiumchlorid (25mM), 3µl dNTPs (2.5mM), 0.5µl Taq DNA Polymerase (2.5 Units, Perkin Elmer)] pipettiert, gemischt und folgenden Thermozyklen unterworfen:
1. Initiale Denaturierung für 1 Minute bei 90°
2. 35 Zyklen, jeweils 28 Sekunden bei 94° C (Denaturierung), 28 Sekunden bei 50° C (Annealing) und 38 Sekunden bei 60° C (Verlängerung)

### 2. PCR

Fünf µl des 1.PCR Ansatzes werden mit 45µl eines 2.PCR Reaktionsgemisches [16.55µl Wasser, 5µl 10x PCR-Puffer (Perkin Elmer), 3µl Magnesiumchlorid (25mM), 4µl dNTPs (2.5mM), 8µl des Primers 3 (10pmol/µl; siehe SEQ ID No. 3 des Sequenzprotokolls), 8µl des Primers 4 (10pmol/µl; siehe SEQ ID No. 4 des Sequenzprotokolls), 0.25µl der TaqMan Sonde 5 (10pmol/µl; siehe SEQ ID No. 5 des Sequenzprotokolls), 0.2µl TaqDNA Polymerase (2.5 Units, Perkin Elmer)] gemischt und folgenden Thermozyklen unterworfen:
1. Initiale Denaturierung für 1 Minute bei 90°
2. 35 Zyklen, jeweils 28 Sekunden bei 94° C Denaturierung und 1 Minute bei 56° C Annealing und Verlängerung
3. Kühlen bei 4° C bis zur Auswertung.

### Auswertung

Die PCR-Reaktion wird in einem Fluoreszenzspektrometer ausgewertet. Dazu wird die Fluoreszenz bei der Reporterwellenlänge (518 nm für FAM) gemessen. Es wird ein Schwellenwert auf Basis der Fluoreszenz von Negativkontrollen, die keine Zielsequenz enthalten, kalkuliert, und Unbekannte werden daran ausgewertet.

### Beispiel 2: HCV nested PCR, Detektion durch Molekular Beacons

### RNA Extraktion

Zum Nachweis von Hepatitis C Virus RNA wird diese zunächst mit Hilfe von Standardverfahren extrahiert (z.B. Ishizawa M., Kobayashi Y., Miyamura T., Matsuma, S: Simple procedure of DNA isolation from human serum. Nucl. Acids Res. 1991; 19:5792). Die Amplifikation wird wie folgt angesetzt:

### cDNA Synthese/1.PCR

Zehn µl der extrahierten RNA werden mit 25µl 2x Reaction Mix (Life Technologies), 2 µl des Primers 6 (50 pMol/µl; siehe SEQ ID No. 6 des Sequenzprotokolls), 2 µl des Primers 7 (50 pMol/µl; siehe SEQ ID No. 7 des Sequenzprotokolls), 10 µl Wasser und 1 µl Superscriptll/Taq Polymerase Mix (Life Technologies) gemischt und folgenden Thermozyklen unterworfen:
1. Inkubation zur cDNA Synthese für 1 Stunde bei 50° C
2. Initiale Denaturierung für 2 Minuten bei 94°
3. 35 Zyklen, jeweils 28 Sekunden bei 94° C (Denaturierung), 28 Sekunden bei 50° C (Annealing) und 38 Sekunden bei 60° C (Verlängerung)

### 2. PCR

Fünf µl des 1.PCR Ansatzes werden mit 45µl eines 2.PCR Reaktionsgemisches [16.55µl Wasser, 5µl 10x PCR-Puffer (Perkin Elmer), 3µl Magnesiumchlorid (25mM), 4µl dNTPs (2.5mM), 8µl des Primers 8 (10pmol/µl; siehe SEQ ID No. 8 des Sequenzprotokolls), 8µl des Primers 4 (10pmol/µl; siehe SEQ ID No. 4 des Sequenzprotokolls), 0.25µl der Molecular Beacon Sonde 9 (5pmol/µl; siehe SEQ ID No. 9 des Sequenzprotokolls), 0.25µl Taq DNA Polymerase (2.5 Units, Perkin Eimer)] gemischt und folgenden Thermozyklen unterworfen:
1. Initiale Denaturierung für 1 Minute bei 90°
2. 35 Zyklen, jeweils 28 Sekunden bei 94° C Denaturierung, 28 Sekunden bei 56° C (Annealing) und 38 Sekunden bei 72° C (Verlängerung)
3. Abkühlen auf 20° C innerhalb von 10 Minuten

### Auswertung

Die PCR-Reaktion wird in einem Fluoreszenzspektrometer ausgewertet. Dazu wird die Fluoreszenz bei der Reporterwellenlänge (518 nm für FAM) gemessen. Es wird ein Schwellenwert auf Basis der Fluoreszenz von Negativkontrollen, die keine Zielsequenz enthalten, kalkuliert, und Unbekannte werden daran ausgewertet.

### Ergebnisse

Sowohl in der Nachweisbarkeit von HCV Genotypen (es wurden Isolate der Genotypen 1 bis 5 getestet) wie auch der analytischen Sensitivität wurden bei Experimenten unter den oben angegebenen Bedingungen im Vergleich mit einer nested PCR mit autologen Primern und Sonden äquivalente Ergebnisse erzielt.

Für das erfindungsgemäße Nukleinsäure-Amplifikationsverfahren ist es nicht erforderlich, dass beide Primer des eingesetzten Primerpaars heterolog sind. Auch durch Kombination eines autologen mit einem heterologen Primer lässt sich eine zum Nachweis bzw. zur Gewinnung der Zielnukleinsäure geeignete Amplifizierung durchführen.

Gegenstand der Erfindung ist auch ein Reagenziensatz zur Durchführung einer PCR, bei dem einer oder beide Primer heterolog sind. Gegenstand der Erfindung ist außerdem ein Reagenziensatz, der zusätzlich zu den vorstehend genannten Primern auch noch eine Oligonukleotid-Sonde enthält, die aus einem Genom eines artfremden Organismus abgeleitet ist, also heterolog ist und vorzugsweise als Molecular Beacon-Sonde vorliegt.

Für die erfindungsgemäßen Oligonukleotide kann die Universalbase Inosin dazu verwendet werden, um etwaige Fehlstellen (mismatches) auszugleichen, die eine vollständige Hybridisierung mit der Nukleotid-Sequenz der Zielnukleinsäure verhindern. Eine unvollständige Hybridisierung mit der Zielnukleinsäure kann auch dadurch entstehen, dass noch unbekannte Varianten der Zielnukleinsäure vorliegen. Diese Varianten können durch die erfindungsgemäßen heterologen Primer und Sonden noch spezifisch erfasst werden, indem die Universalbase Inosin an der Fehlstelle in den Primer oder die Sonde eingefügt wird. Das erfindungsgemäße Verfahren hat somit eine größere Nachweisbreite als ein Verfahren, das ausschließlich mit autologen Primern und Sonden arbeitet.

SEQUENZPROTOKOLL

<110> Aventis Behring GmbH

<120> Verfahren zum Auffinden von Oligonukleotid-Sequenzen
      für Nukleinsäure-Amplifikationsverfahren

<130> 2000/A006-A3b

<140>
<141>

<160> 9

<170> PatentIn Ver. 2.1

<210> 1
<211> 27
<212> DNA
<213> Red sea bream iridovirus gene

<400> 1
ggticaiggt ctaigagaci icccggg                                    27

<210> 2
<211> 18
<212> DNA
<213> Homo sapiens

<400> 2
actccaccat agatcact                                              18

<210> 3
<211> 22
<212> DNA
<213> Homo sapiens

<400> 3
ctaiccatgg cittagtatg ag                                                22

<210> 4
<211> 21
<212> DNA
<213> Human DNA sequence from PAC 388M5

<400> 4
agcaccctit caggcagtac c                                                 21

<210> 5
<211> 20
<212> DNA
<213> Helianthus tuberosus sst-1 gene

<400> 5
FAM-tgggtcicga aagiccttgt-TAMRA                                         20

<210> 6
<211> 25
<212> DNA
<213> Drosophila melanogaster

<400> 6
gctcatgitg cacgiictic gagac                                             25

<210> 7
<211> 20
<212> DNA
<213> cyprinella galactura

<400> 7
catagitcac tcccctgtga                                                    20

<210> 8
<211> 25
<212> DNA
<213> Bovine viral diarrhea virus

<400> 8
aaagigicta gccatgicit tagta                                              25

<210> 9
<211> 31
<212> DNA
<213> Helianthus tuberosus sst-1 gene

<400> 9
FAM-gtacctgggt cicgaaagic cttgtggtact-TAMRA                              31

### Annex zu den Anmeldungsunterlagen - nachgereichtes Sequenzprotokoll

SEQUENZPROTOKOLL

<110> Aventis Behring GmbH

<120> Verfahren zum Auffinden von Oligonukleotid-Sequenzen für
Nukleinsäure-Aplifikationsverfahren

<130> 2000/A006-A3b

<160> 9

<170> PatentIn Ver. 2.1

<210> 1
<211> 27
<212> DNA
<213> Red sea bream iridovirus gene
<220>
<221> modified_base
<222> 4, 7, 14, 20, 21
<223> /mod_base = a=i

<400>	1
ggtacaaggt ctaagagaca acccggg	27

<210>	2
<211>	18
<212>	DNA
<213>	Homo sapiens

<400>	2
actccaccat agatcact	18

<210>	3
<211>	22
<212>	DNA
<213>	Homo sapiens
<220>
<221>	modified_base
<222>	4, 12
<223>	/mod_base = a=i

<400>	3
ctaaccatgg cattagtatg ag	22

<210>	4
<211>	21
<212>	DNA
<213>	Homo sapiens
<220>
<221>	modified_base

<222>	9
<223>	/mod_base = a=i

<400>	4
agcaccctat caggcagtac c	21

<210>	5
<211>	20
<212>	DNA
<213>	Helianthus tuberosus set 1 gene
<220>
<221>	modified_base
<222>	7, 14
<223>	/mod_base = a=i
<220>
<221>	misc_feature
<222>	1
<223>	/note = FAM coupled to 5' end
<220>
<221>	misc_feature
<222>	20
<223>	/note = TAMRA couples to 3' end

<400>	5
tgggtcacga aagaccttgt	20

<210>	6
<211>	25
<212>	DNA

<213>	Drosophila melanogaster
<220>
<221>	modified_base
<222>	15, 16, 19
<223>	/mod_base = a=i

<400>	6
gctcatgatg cacgaactac gagac	25

<210>	7
<211>	20
<212>	DNA
<213>	Cyprinella galactura
<220>
<221>	modified_base
<222>	6
<223>	/mod_base = a=i

<400>	7
catagatcac tcccctgtga	20

<210>	8
<211>	25
<212>	DNA
<213>	Bovine viral diarrea virus
<220>
<221>	modified_base
<222>	5, 7, 17, 19
<223>	/mod_base = a=i

<400>	8
aaagagacta gccatgacat tagta
25

<210>	9
<211>	31
<212>	DNA
<213>	Helianthus tuberosus sst-1 gene
<220>
<221>	modified_base
<222>	9
<223>	/mod_base = a=i
<220>
<221>	modified_base
<222>	11, 19
<223>	/mod_base = a=i
<220>
<221>	misc_feature
<222>	1
<223>	/note = FAM coupled to 5' end
<220>
<221>	misc_feature
<222>	31
<223>	/note = TAMRA coupled to 3' end

<400>	9
gtacctgggt cacgaaagac cttgtggtact	31

## Patentansprüche

1. Verfahren zum Auffinden von heterologen Oligonukleotid-Sequenzen für ein Nukleinsäure-Amplifikationsverfahren, **dadurch gekennzeichnet, dass**
a) durch Fragmentierung konservierter Bereiche der zu amplifizierenden Zielnukleinsäure sich gegenseitig überlappende Oligonukleotid-Sequenzen erzeugt,
b) diese Sequenzfragmente zum Auffinden ähnlicher DNA-Abschnitte in der Genbank oder anderen DNA-Datenbanken benutzt und damit geeignet heterologe Oligonukleotid-Sequenzen artfremder Organismen ermittelt, und
c) die aufgefundenen heterologen Oligonukleotid-Sequenzen als Primer und/oder Sonden zur Gewinnung der Zielnukleinsäure nach einem Nukleinsäure-Amplifikationsverfahren eingesetzt werden.

2. Verfahren nach Anspruch 1, **dadurch gekennzeichnet, dass** durch Fragmentierung konservierter Bereiche eines Genoms eines Virus sich gegenseitig überlappende, 30 bis 50 Basen umfassende Oligonukleotid-Sequenzen erzeugt und in einer Genbank heterologe Oligonukleotid-Sequenzen ermittelt werden, die zum Nachweis des Virus geeignet sind.

3. Verfahren nach den Ansprüchen 1 bis 2, **dadurch gekennzeichnet, dass** die in den aufgefundenen hybridisierenden, heterologen Oligonukleotid-Sequenzen enthaltenen Fehlstellen (mismatches) durch eine Universalbase (z.B. Inosin) ersetzt und damit eine vollständige Hybridisierung mit der Nukleotid-Sequenz der Zielnukleinsäure erreicht wird.

4. Verfahren zur Nukleinsäure-Amplifikation, **dadurch gekennzeichnet, dass** man die nach den Ansprüchen 1 bis 3 gewonnenen heterologen Oligonukleotid-Sequenzen als Primer und/oder Sonden zur selektiven Gewinnung einer vorbestimmten Zielnukleinsäure einsetzt.

5. Verfahren nach Anspruch 4, **dadurch gekennzeichnet, dass** zur Amplifikation der Zielnukleinsäure ein Nukleinsäure-Amplifikationsverfahren wie die Polymerasekettenreaktion (PCR), NASBA (=Nucleic Acid Sequence-Based Amplification), TMA (Transcription Mediated Amplification) oder LCR (Ligase Chain Reaction) eingesetzt wird.

6. Reagenziensatz zur Durchführung einer Polymerasekettenreaktion, **dadurch gekennzeichnet, dass** er ein Paar Oligonukleotid-Primer enthält, die die gesuchte DNA-Sequenz aufweist, die aus einem Genom eines artfremden Organismus abgeleitet worden ist.

7. Reagenziensatz nach Anspruch 6, **dadurch gekennzeichnet, dass** er zusätzlich eine Oligonukleotid-Sonde enthält, die eine heterologe, aus einem Genom eines artfremden Organismus abgeleitete DNA-Sequenz enthält und mit der von den Primern flankierten DNA-Sequenz der Zielnukleinsäure hybridisiert.

8. Reagenziensatz nach Anspruch 7, **dadurch gekennzeichnet, dass** die Sonde in 5'- und in 3'-Stellung zwei Fluoreszenzfarbstoffe (Reporter und Quencher) trägt, die sich in ihrer Fluoreszenz gegenseitig beeinflussen.

9. Reagenziensatz nach Anspruch 6, **dadurch gekennzeichnet, dass** ein Primer eingesetzt wird, der mit zwei Fluoreszenzfarbstoffen (Reporter und Quencher) markiert ist und dieser Primer mit der zu amplifizierenden DNA-Sequenz am 3'-Ende nicht vollständig hybridisiert.
